# EUROPEAN PATENT APPLICATION

(11) **EP 4 667 085 A1**
(43) Date of publication of application: **24.12.2025**
(21) Application number: 25166750.7
(22) Date of filing: 27.03.2025
(51) Int. Cl.: B01D 53/14, B01D 19/00, B01D 53/26, C12M 1/00, C10L 3/10

(54) **A PROCESS OF CARBON DIOXIDE DESORPTION AND BIOMETHANE RECOVERY WITH BIOGENIC CARBON DIOXIDE**

(30) Priority: 28.03.2024 IN 202421025227
(71) Applicant: Ovee Consulting Engineers, 411041 Pune, Maharashtra (IN)
(72) Inventor: KADU, Bharat Ashok, 411041 Pune, Maharashtra (IN)
(74) Representative: TBK

(57) **Abstract**

The present invention is related to the process of desorption of carbon dioxide from water without any stripping medium such as air or any process gas and further recovery of biogenic carbon dioxide and bio methane for Biogas purification. The production of bio methane and biogenic carbon dioxide is carried out without using air for desorption and using counter-current method. Another aspect of the present invention is the process involves compression of partially desulfurized raw biogas containing hydrogen sulfide and to use chilled water from the top of the column while the gas is introduced from the bottom with absorption column as a twin bed unit where each bed has a height of 2-6 meters with twin bed packed type vessel ranging from ¼ to 2 inches in size to obtain bio methane containing 90-99% methane to achieve the desired water dew point, complying with the fuel standards of the region.

## Description

### FIELD OF THE INVENTION

The invention relates to the process of desorption of carbon dioxide from water without any stripping medium such as air or any process gas and recovery of biogenic carbon dioxide and bio methane for Biogas purification.

### BACKGROUND OF THE INVENTION

Biogas production has been around since ancient civilizations and was used for heating and lighting purposes. In modem times, biogas has emerged as a promising biofuel and an alternative to natural gas on a global scale. Biogas can be generated from various sources such as animal dung, agricultural residues, energy crops, industrial waste, and municipal solid waste.

The typical composition of raw biogas is as below:
1. Methane: 50-65% v/v
2. Carbon Dioxide: 35-45 % v/v
3. Moisture: Saturated at gas temperature
4. Hydrogen Sulfide: 0-4% v/v
5. Oxygen, Nitrogen, Ammonia and other hydrocarbons in traces.

The purified biogas are named as Biomethane, Synthetic Natural Gas (SNG), Renewable Natural Gas (RNG), Bio CNG, Compressed Biogas etc across the globe which are mostly opted as potential alternate vehicle fuels. For use as vehicle fuel, the raw biogas needs to be further purified for enhancement of the methane content from 50-65% v/v to about 90-99% v/v in compliance with local regulations.

The main component which has relatively high energy content is the methane (CH₄) gas which can be used as fuel. However, the percentage of methane content is relatively low in raw biogas which becomes an issue for vehicular applications as that can reduce the heat value and interferes with the combustion process. The enhancement of methane is done via a series of purification steps where generally the hydrogen sulphide (H₂S) is removed first followed by the removal of the rest of the impurities as applicable.

There are numerous existing technologies available for biogas purification each with associated advantages and limitations.

Out of the various alternatives, one of the solutions to reduce the content of carbon dioxide (CO₂) is the water-scrubbing method. High-pressure water scrubbing is a popular method used worldwide for the purification of biogas in which a greener solvent water is used which is environment friendly and readily available at lower or sometimes at no cost. The water-scrubbing is based on the physical effects of dissolving gas in water medium. In the same process, carbon dioxide (CO₂) and hydrogen sulphide (H₂S) within the biogas will be absorbed by water because of the greater solubility of these gases in water than methane gas (CH₄). The water-scrubbing method is carried out using a column in which the absorption process will occur. Biogas is inserted at high pressure from the bottom of the water scrubber column while a controlled flow of water is streamed from the top of the scrubber column, producing a counter current flow scrubbing process.

The conventional process followed for biogas purification using high pressure water scrubbing is as follows:
1. The raw biogas is compressed to a pressure of 6-10 Bar A (*Bar A is 'Absolute Pressure' defined as the pressure measured with reference to full* vacuum *or no atmospheric pressure*) using either a screw or a reciprocating compressor.
2. The compressed gas is then passed to the absorption column where it is contacted with chilled water at a temperature of 10-20 degrees Celsius in a counter-current method. This causes the carbon dioxide and hydrogen sulphide to dissolve in the water to an equivalent solubility at the given temperature and pressure.
3. The exhaust from the absorption column is clean gas which generally complies with local fuel specification norms after dehumidification.
4. The gas-laden liquid stream is then flashed at a pressure of 2-6 Bar A to recover methane. The flashed gas stream is recycled back to the compressor while the residual liquid stream goes for further desorption in the desorption column.
5. In the desorption column, the liquid from the flash vessel is further flashed to ambient pressure and then contacted with air in a counter-current method. The air strips out the residual dissolved gases so that the regenerated liquid can be recycled back to the absorption column for further absorption or abatement of biogas impurities.

The above process is configured in innovative ways in various prior arts but has the following few issues associated to solve:
1. The Methane loss during the process is as high as 10% if not more.
2. The exhaust stream from the desorption column is always contaminated with air which limits the recovery of the biogenic Carbon dioxide (CO₂) as against other methods of biogas up gradation like Pressure swing adsorption, membrane and amine etc.
3. Traces of air ingress in the Bio-Methane due to regeneration or desorption of gases by air stripping method.

### OBJECTIVES OF THE INVENTION

The principal object of the present invention is to develop a process for desorption of carbon dioxide from water without the requirement of stripping medium air or any other process gas and specifically recovery of biogenic carbon dioxide and bio methane from water scrubbing process.

Another object of the present invention is to involve the flashing of gas-rich water steam at medium pressure for maximum bio methane recovery with the recycling of the vent stream back to the suction of the compressor.

Another object of the present invention is to achieve overall methane recovery is up to 99% with only 1.0 % methane slip at rated gas loadings.

Another object of the present invention is to water after vacuum flash vessel or sub atmospheric pressure flash vessel is pumped to the absorption column for further absorption of carbon dioxide and hydrogen sulfide from compressed biogas.

Another object of the present invention is to wet bio methane undergoes molecular sieve type drying wherein the process involves regeneration with heating element to achieve a specific moisture dew point in the dry bio methane.

Yet another object of the present invention is the gas desorption carried out in a series of flashing stages from medium pressure to vacuum without any stripping medium such as air or process gas to avoid tpotential ontamination of the bio methane and biogenic carbon dioxide with Air or process gas as applicable.

These and other objectives of the present invention will be apparent from the drawings and descriptions herein. Every object of the invention is attained by at least one embodiment of the present invention.

### SUMMARY OF THE INVENTION

A summary is provided herein to facilitate an understanding of the innovative characteristics unique to the disclosed embodiments and is not intended for the full description of the invention. In accordance, a full appreciation of various aspects of the preferred embodiments provided herein can be understood in detail by taking the entire specification, claims, drawings, and abstract as a whole.

An aspect of the present invention is to device processes for the production of Biomethane and Biogenic carbon dioxide without using air for desorption by counter-current method.

Another aspect of the present invention is the process involves the compression of partially desulfurized raw biogas using a screw or reciprocating compressor.

Another aspect of the present invention is to use chilled water at a temperature range of 5-20°C from the top of the column while the gas is introduced from the

Bottom such that the gas and water are contacted in a counter-current way with liquid to gas ratio of 1.0 to 1.3 m³/m³ resulting in dissolution of carbon dioxide and hydrogen sulfide in water in relation to their solubility at a particular pressure and temperature.

Another aspect of the present invention is to use the absorption column as a twin bed unit where each bed has a height of about 2-6 meters with twin bed packed type vessel that utilizes packing media ranging from ¼ to 2 inches in size.

Another aspect of the present invention is to effectively absorb the carbon dioxide and hydrogen sulfide from the biogas equipped with liquid and gas distributors, as well as redistributor arrangements to effectively absorb the carbon dioxide and hydrogen sulfide from the biogas.

Another aspect of the present invention is to obtain bio methane containing 90-99% methane which undergoes dehumidification using dedicants to achieve the desired water dew point, complying with the fuel standards of the region.

Another aspect of the present invention is that the method of the present invention employs gas solubility differences to separate impurities from biogas.

### BRIEF DESCRIPTION OF THE DRAWINGS

The drawings described herein are for illustrative purposes only of selected embodiments and not all possible implementations are not intended to limit the scope of the present disclosure. Further objectives and advantages of this invention will be more apparent from the ensuing description when read in conjugation with the accompanying drawing, as detailed below:

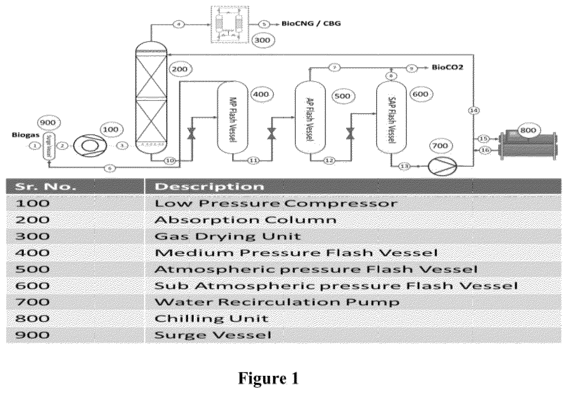

**Fig. 1** shows the flow chart of the process of carbon dioxide desorption and bio methane recovery with biogenic carbon dioxide.

### REFERENCE NUMERALS FOR THE DEVICE AND THE PARTS THEREIN

1. Partially desulfurized raw biogas (1).
2. Mixed stream of gas (2).
3. Compressed raw biogas stream (3).
4. Methane Rich Stream (4)
5. Biomethane Stream (5).
6. First flash vessel vent stream (6).
7. Second flash vessel vent stream (7).
8. Third flash vessel vent stream (8)
9. Second and third flash vessel mixed vent stream (9).
10. Gas rich water (10).
11. Liquid stream (11).
12. Water fraction (12)
13. Gas lean water stream (13).
14. High pressure water stream (14).

### DETAILED DESCRIPTION OF THE INVENTION

The following description includes the best known method as one of the embodiments of the present invention. It will be clear from this description of the invention that the invention is not limited to the illustrated embodiments but the invention also provides a scope for a variety of modifications and embodiments thereto. Therefore, the present description should be seen as illustrative and not limiting. While the invention is susceptible to various modifications, it should be understood, that there is no intention to limit the invention, alternative constructions, and equivalents falling within the spirit and scope of the invention as defined in the claims.

In any embodiment described herein, the open-ended terms "comprising," "comprises," and the like (which are synonymous with "including," "having," and "characterized by") may be replaced by the respective partially closed phrases "consisting essentially of," consists essentially of," and the like or the respective closed phrases "consisting of," "consists of, and the like.

As used herein, the singular forms "a", "an" and "the" designate both the singular and the plural, unless expressly stated to designate the singular only.

An embodiment of the present invention is herein developed a process for producing Biomethane and Biogenic carbon dioxide without using air for desorption as described below:
The process begins with the compression of partially desulfurized raw biogas (1) containing 10-500 ppmv hydrogen sulfide using a screw or reciprocating compressor (100). The compressed biogas (3) is then fed to the absorption column (200) which is a twin bed packed type column comprising packing media ranging from ¼ to 2 inches in size and is equipped with liquid and gas distributors as well as redistributor arrangements to effectively absorb the carbon dioxide and hydrogen sulfide from the biogas using chilled water (14) in a temperature range of 5-20°C, precisely of 15°C which is then sprayed from the top of the column while biogas gas (3) is introduced from the bottom.

Another embodiment of the present invention discloses the role of counter-current principle. The gas and water are contacted in a counter-current way with liquid to gas ratio of 1.0 to 1.3 m³/m³ to be more precise 1.1 m³/m³ resulting in the dissolution of carbon dioxide and hydrogen sulfide in water in relation to their solubility at a particular pressure and temperature. Biomethane (4) containing 90-99% methane undergoes dehumidification (300) using dedicants to achieve the desired water dew point complying with the fuel standards as per the region. The bio methane (5) is compressed to the desired pressure to suit the end application of Grid injection, Cascade storage or dispensing etc. The gas-rich water (10) from the absorption column is then subjected to medium pressure flash (400) at 2-5 Bar A, precisely at (3.5 to 4.5 Bar A) at rated load and at (1.5 to 2.0 Bar A) reduced plant capacity to recover the dissolved methane. The flashed gas (6) is recycled back to the compressor suction limiting methane slip to a maximum of 1% at all operating loads of the plant.

The partially flashed gas-laden water (11) from the medium pressure flash vessel is subjected to an atmospheric pressure flash vessel (500) where most of the Carbon dioxide get removed from the water as a vent (7). The water fraction is subjected to vacuum flash at 0.1 to 0.3 Bar A, particularly at 0.2 Bar A using a liquid ring or dry-type vacuum pump in sub atmospheric flash vessel (600). The vent (8) from the sub-atmospheric flash vessel (600) is mixed with atmospheric flash vessel (500) vent stream (7) to form a mixed vent stream (9). The mixed vent stream (9) is 98-99.5% biogenic carbon dioxide and balances methane gas on moisture-free basis. This stream (9) can be further subjected to industrial grade and/or food grade quality carbon dioxide recovery via cryogenic separation which is not a part of this embodiment.

Another embodiment of the present invention illustrates gas desorption which occurs in a series of flashing from medium pressure to vacuum such that the process avoids contamination of biogenic carbon dioxide with air and prevents ingress of air in bio methane by performing gas desorption without Air stripping. The gas lean water (13) is then pressurised using the centrifugal pump (700) to form high pressure water stream (14) which goes to absorption column (200) for further absorption of contaminants from Biogas to form methane rich stream (4) called Biomethane and the cycle continues. The water temperature shall be maintained using chiller (800) to compensate for the heat addition while pumping, compression, ambient conditions etc.

Another embodiment of the present invention discloses the principle behind use of three flash vessels namely:
**MP Flash Vessel:** Medium flash vessel is used mainly for Methane recovery.
**AP Flash Vessel:** Low opex Gas stripping stage
**SAP Flash Vessel:** Vacuum Gas Stripping stage for requisite Regeneration of absorption liquid.

The process is based on Henry's law which states that at the amount of gas that is dissolved in a liquid is directly proportional to the partial pressure of that gas above the liquid when the temperature is kept constant. Based on Henry's law, the absorption column for the present invention is selected to operate at 9 Bar A Pressure and 15° C temperature with adequate water flow rate to maximum possible gases which could dissolve at given set of operating conditions. As per henry's law the solubility of gases goes down with pressure and hence in reverse the additional gases which have been absorbed at higher pressure comes out from the water. Once absorption gets completed in the next step it is desorbed so that the same water can be recycled back for additional gas absorption. Though the solubility of hydrogen sulfide and carbon dioxide is higher as against methane, some of the methane get trapped in the liquid which needs to be recovered back to achieve less than 1 % methane loss. In the present invention, medium pressure flash at 4.0 Bar A so reduction of liquid pressure from 9.0 Bar A to 4.0 Bar A reduced the solubility of gases and quantity of gases above saturation to a gaseous phase which is recycled back to compressor suction for methane recovery.

Once methane is recovered ideally one can go for a single stage sub-atmospheric flash i.e. vacuum flash but we found out that about 70-75% of the gaseous load flashes off when the liquid is exposed to atmospheric pressure which otherwise consumed significant power for vacuum pump operation there by impacting opex and opex of the system. For optimization of the opex we added the atmospheric flash stage which reduced the electricity requirement apart from capital expenditure savings.

One of the highlighting inventive step of the present invention is the concept of 'Sub Atmospheric Flash' as it reduces the residual gas content to the optimal levels which in conventional approaches is done with 'Air stripping' which adversely impact the quality of the biogenic carbon dioxide, moreover, some of the dissolved air also contaminates the produced bio methane as operating under vacuum instead of any stripping medium reduces the partical pressure and the solubility of target gases in liquid medium air or any other process gases

**Fig. 1** shows a flow chart of the step wise process followed to achieve the desired results comprises the detailed steps of:
1. Get raw biogas at the battery limit of the said specification as mentioned in state of the art to compressor surge vessel (900).
2. Compressor surge vessel (900) also receives 'flash stream' and the combined stream is passed on to the absorption column bottom.
3. In absorption column, liquid flow rate is about 1.1 times the actual volumetric flow rate of the gas which then further contacted counter currently to absorb the gases in accordance with their solubility at 9 bar A pressure and 15°C temperature.
4. The Clean gas from the top of the column is sent to the drying unit equipped with an adequate system to achieve the desired moisture level and the final product is Biomethane after drying.
5. The liquid rich in gases are then subjected to a medium pressure flash at 4.0 Bar A pressure and the fraction of gases obtained are sent to the compressor surge vessel for methane recovery while the liquid fraction is subjected to further regeneration steps.
6. The liquid from medium flash vessel shall be subjected to twin stage flash first at Atmospheric pressure where about 70 to 75% of the gas loading get removed and then to vacuum at 0.2 bar A where balance gas load get removed via vacuum pump.
7. The liquid from sub atmospheric flash vessel is then pumped back to the absorption column for further absorption of gases periodically.
8. The small pre-decided fraction of the liquid from the sub atmospheric flash vessel or atmospheric flash vessel is then passed through the chiller unit to maintain the temperature.
9. All equipment as applicable shall be adequately insulated to avoid heat gain from atmospheric conditions across the season.
10. Absorption column and the flash vessels shall have adequate instrumentation which shall be flexible enough to adjust to the varying operating conditions and requirements. Ideally the turn down requirement pressure loss shall be through restriction orifice while rated condition control shall be left to control valves.

Another embodiment discloses the optimization of parameters to select appropriate working conditions.

Numerous Insilco analysis to arrive at a right set of operating parameters with some key evaluation factors like Overall Methane recovery, Biomethane Concentration, the Saturation level of liquid with respect solubility data.

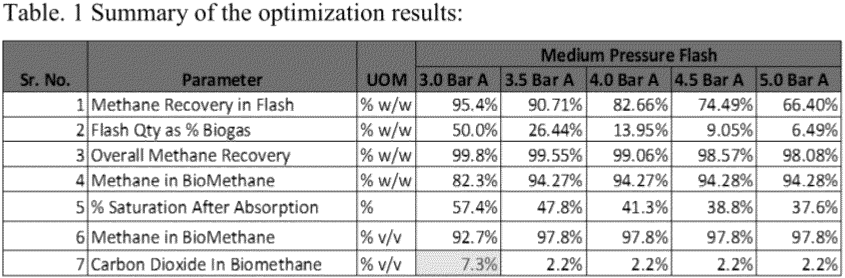

The above table shows as the medium flash pressure goes up the overall methane recovery as a function of methane recovery in flash stream goes down, however the bio methane specification remain unchanged and the quantity of flash gases goes down. Below 3.5 Bar A, there is a significant deterioration in product specification but the maximum methane recovery. So considering the data we selected 4.0 Bar A as the key operating parameter for medium pressure flash.

It is to be noted that the operating pressure is kept constant at 9 Bar A as the STD compressor suffice the duty. At lower operating pressure liquids the absorption capacity goes down thereby impacting the overall system performance and opex due to higher equipment sizes are required on account of less dens gas flow and higher water recirculation.

The operating temperature is always preferred to be low and we selected 15°C as this will be maintained by chilling water which has supply temperature of 10°C to get 15°C in the absorption circuit. It is also possible to further lower the temperature but then chilling circuit need specialized recirculation fluid due to water freezing issues. The operating parameters are selected so as to keep tap on overall cost of ownership.

Another embodiment of the present invention discloses about one working example as illustrated below:

### Example 1

About 1000 sm³/hr of the pre-desulfurized raw biogas (1) and medium pressure flash vessel vent stream (6) forming mixed stream (2) is compressed to 9 Bar A using biogas compressor (100) to for compressed biogas stream (3).

The typical composition of raw biogas is as below,
Methane: 54.13% v/v
Carbon Dioxide: 39.7% v/v
Hydrogen Sulfide: 100 ppmv
Moisture: 6.2 % v/v
Temperature: 37 degree Celsius.

The 146 m³/hr of Gas lean water (14) and compressed biogas stream (3) of 137 Am³/hr contacted in absorption column (200) to form wet bio methane stream (4) of about 407 kg/hr which contains about 97.67% v/v. Methane with 383.60 kg/hr of Methane out of 386.39 kg from feed. The wet bio methane stream (4) and then subjected to dehumidification (300) to generate Biomethane Stream (5). Methane recovery of about 99.28% while hydrogen sulfide of 11 mg/sm³ with 95.8% hydrogen sulfide separation.

The Gas rich water (10) from the Absorption column (200) and then flashed in medium pressure flash vessel (400) to generate first vent stream (6) of 207 kg/hr which contains 19.3% of methane and 79.7% of carbon dioxide which gets recycled to the compressor (100) suction for methane recovery. This step also generates the liquid stream (11) which is then subjected to atmospheric flash in an atmospheric pressure flash vessel (500) where a second vent stream (7) of 566 kg/hr is generated 97% v/v carbon dioxide & rest of water and methane. The liquid (12) from the atmospheric flash vessel (500) is then subjected to a vacuum flash in a sub atmospheric flash vessel (600) to generate third vent stream (8) of about 204 kg/hr and Gas lean water stream (13). The Third vent stream contains about 91.4 % v/v Carbon dioxide, 8.5 % v/v water and traces gases like methane and Hydrogen sulfide. The gas lean water stream (13) is pressurized using centrifugal pump (700) to form high pressure gas lean water stream 14 which is sent to absorption column(200) for further absorption of gases. The vacuum generation in sub atmospheric flash vessel is done using liquid ring type or dry type vacuum pump or jet type vacuum device.

Second and third vent streams combine to form mixed vent stream (9) contains about 97% Carbon dioxide, 2.3% water and rest are trace gases like methane and hydrogen sulphide.

Another embodiment of the present invention discloses the 'workable range' of the parameters within which the best method described can be worked out.

### A. At rated / design capacity:

Absorption Columns - 9.0 Bar A and 15°C and Liquid to Gas ratio of 1.1
MP Flash - 4.0 bar A
AP flash -15°C
SAP Flash - 0.2 Bar A and 15°C.

### B. At 67% Turn down operation:

Absorption Columns - at 9.0 Bar A and 15°C
Liquid Flow rate: Identical to rated capacity
MP Flash - 2.0 bar A
AP flash -15°C
SAP Flash - 0.2 Bar A and 15°C

We can go down further in 'MP Flash' but it disturbs hydraulic balance of the system as we took advantage of this to save on pumping power.

### ADVANTAGES OF THE PRESENT INVENTION

An advantage of the present invention is to develop a process of desorption of carbon dioxide from water without any a stripping medium like air or any other process gas and recovery of biogenic carbon dioxide and bio methane.

Another advantage of the present invention is the developed process is for the production of Biomethane and Biogenic carbon dioxide without using air for desorption by counter-current method.

Another advantage of the present invention is the 98 to 99.5 % v/v Biogenic carbon dioxide recovery on moisture free basis with the top of absorption column wet bio methane is recovered with 90- 99% v/v methane content.

Another advantage of the present invention is that the flashed gas is recycled back to the compressor suction thereby limiting the methane slip of the process to a maximum of 1% at all operating loads of the plant.

Another advantage of the present invention is the water after vacuum flash vessel or sub atmospheric pressure flash vessel is pumped to the absorption column for further absorption of carbon dioxide and hydrogen sulfide from compressed biogas.

Another advantage of the present invention is the water after vacuum flash vessel or sub atmospheric pressure flash vessel is pumped to the absorption column for further absorption of carbon dioxide and hydrogen sulfide from compressed biogas.

## Claims

1. A process of carbon dioxide desorption and bio methane recovery with biogenic carbon dioxide comprising the steps of :
(a) taking pre-desulfurized raw biogas;
(b) compressing the biogas using screw or reciprocating type compressor to form a compressed raw biogas stream of step (a);
(c) contacting the compressed biogas stream obtained in step (b) counter currently with water in the absorption column;
(d) generating first stream of wet biogas;
(e) drying the wet bio methane obtained in step (d) using a molecular sieve dehumidification to regenerate heating element and to achieve specific moisture dew in dry bio methane;
(f) point in the dry biomethane meet fuel specifications;
(g) flashing the gas rich water obtained in step (c) to a medium pressure for bio methane recovery in a medium pressure flash vessel;
(h) recycling back the vent or flashed gases obtained in step (f) to a compressor suction;
(i) subjecting the water fraction from the medium pressure flash vessel of step (f) to twin stage flask at atmospheric pressure and sub atmospheric pressure to generate the second stream of biogenic carbon dioxide; and
(j) pumping the residual water stream to the absorption column for further absorption of gases.

2. The process of carbon dioxide desorption and bio methane recovery as claimed in claim 1, wherein the process temperature is maintained using chiller unit.

3. The process of carbon dioxide desorption and bio methane recovery as claimed in claim 1, wherein the pre-desulfurized raw biogas is obtained from agricultural biomass, energy crops, municipal solid waste, animal waste, industrial waste and food waste.

4. The process of carbon dioxide desorption and bio methane recovery as claimed in claim 1, wherein the compressing of the Biogas and the medium pressure flash vessel vent is at a pressure range of 6 to 10 Bar A.

5. The process of carbon dioxide desorption and bio methane recovery as claimed in claim 1, wherein the contacting of the compressed biogas stream with water in the absorption column is at a temperature range of 5°C to 20°C.

6. The process of carbon dioxide desorption and bio methane recovery as claimed in claim 1, wherein the absorption column for the counter-current contacting of the compressed bio gas stream is a twin bed packed column. With 1/4 to 2.0 inch packing medium with the height of each bed ranging from 2 meter to 6 meter.

7. The process of carbon dioxide desorption and bio methane recovery as claimed in claim 1, wherein the recycling back of the flashed gases to a compressor suction is at a pressure range of 2 Bar A to 5 Bar A for bio methane recovery.

8. The process of carbon dioxide desorption and bio methane recovery as claimed in claim 1, wherein the absorption column for the counter-current contacting of the compressed bio gas stream is equipped with liquid and gas distributors & re-distributors.

9. The process of carbon dioxide desorption and bio methane recovery as claimed in claim 1, wherein the recycling back of the flashed gases to a compressor suction is at a pressure range of 2 Bar A to 5 Bar A for bio methane recovery.

10. The process of carbon dioxide desorption and bio methane recovery as claimed in claim 1, wherein the flashing of the water fraction from the medium pressure flash vessel to the twin stage flash where a two stage flashing at atmospheric pressure and at vacuum of 0.1 to 0.3 Bar A.
